# EUROPÄISCHE PATENTANMELDUNG

(11) **EP 4 060 015 A2**
(43) Veröffentlichungstag der Anmeldung: **21.09.2022**
(21) Anmeldenummer: 22161934.9
(22) Anmeldetag: 14.03.2022
(51) Int. Cl.: C12M 1/107, C12M 1/36, C12M 1/00, F24D 3/08, F24D 11/00, F24D 17/00, F24F 11/00, G06F 1/20, H04L 12/46, H05K 7/20

(54) **RECHENZENTRUMSEINRICHTUNG UND VERFAHREN ZUM BETREIBEN EINER RECHENZENTRUMSEINRICHTUNG**

(30) Priorität: 16.03.2021 DE 102021106416
(71) Anmelder: bioplusmining GmbH, 75196 Remchingen (DE)
(72) Erfinder: Lichtblau, Matthias, 65779 Kelkheim (DE); Rizov, Boris Borisov, 76227 Karlsruhe (DE); Koeck, Oliver, 75196 Remchingen (DE); Erich Walz, Peter Fritz, 76456 Kuppenheim (DE); Schuller, Rainer Hermann, 75175 Pforzheim (DE); Wessner, Ulf Martin, 72461 Albstadt (DE)
(74) Vertreter: RPK Patentanwälte Reinhardt, Pohlmann und Kaufmann Partnerschaft mbB

(57) **Zusammenfassung**

Die Erfindung geht aus von einer Rechenzentrumseinrichtung (100) und ein Verfahren zum Betreiben einer Rechenzentrumseinrichtung (100) mit einer ersten Quelle (10) zur Erzeugung von Biogas, einer zweiten Quelle (12) zur Erzeugung von elektrischem Strom aus dem Biogas, die mit der ersten Quelle (10) wirkverbunden ist und zum Betreiben mit Biogas der ersten Quelle (10) ausgebildet ist. Ferner weist die Rechenzentrumseinrichtung (100) eine erste Senke (20) auf, die wenigstens ein Rechenzentrum (24) mit elektrischen Rechenanlagen (22) zur Nutzung des von der zweiten Quelle (12) bereitgestellten elektrischen Stroms umfasst. Weiterhin weist die Rechenzentrumseinrichtung (100) eine erste Kühleinrichtung (30) auf, die mit der ersten Senke (20) wirkverbunden ist und zur Abfuhr von Abwärme aus der ersten Senke (20) ausgebildet ist. Des Weiteren weist die Rechenzentrumseinrichtung (100) eine zweite Senke (40) auf, die mit der ersten Senke (20) wirkverbunden ist und zur Abfuhr von Abwärme aus der ersten Senke (20) ausgebildet ist.

## Beschreibung

Die Erfindung betrifft eine Rechenzentrumseinrichtung und ein Verfahren zum Betreiben einer Rechenzentrumseinrichtung

### Stand der Technik

Es ist bekannt, die Abwärme von Rechenzentren zu nutzen, indem diese in Fernwärmenetze eingespeist wird und in daran angeschlossenen Kommunen beispielsweise zu Heizzwecken genutzt wird. Weiterhin bekannt ist, Heizsysteme auf Basis von Rechnern auf Gebäude zu verteilen und so die Abwärme der Rechner, die direkt in den Gebäuden entsteht, dort zu verwerten. Eine solche Nutzung ist beispielsweise in der DE 102011000444 B4 beschrieben.

### Offenbarung der Erfindung

Eine Aufgabe der Erfindung ist die Schaffung einer Rechenzentrumseinrichtung mit einer verbesserten Energienutzung.

Eine weitere Aufgabe der Erfindung ist die Schaffung eines Verfahrens zum Betreiben einer solchen Rechenzentrumseinrichtung mit einer verbesserten Energienutzung.

Die Aufgaben werden durch die Merkmale der unabhängigen Ansprüche gelöst. Günstige Ausgestaltungen und Vorteile der Erfindung ergeben sich aus den weiteren Ansprüchen, der Beschreibung und der Zeichnung.

Die in den Patentansprüchen einzeln aufgeführten Merkmale sind in technologisch sinnvoller Weise miteinander kombinierbar und können durch erläuternde Sachverhalte aus der Beschreibung und durch Details aus den Figuren ergänzt werden, wobei weitere Ausführungsvarianten der Erfindung aufgezeigt werden.

Es wird eine Rechenzentrumseinrichtung vorgeschlagen mit einer ersten Quelle zur Erzeugung von Biogas; einer zweiten Quelle zur Erzeugung von elektrischem Strom aus dem Biogas, die mit der ersten Quelle wirkverbunden ist und zum Betreiben mit Biogas der ersten Quelle ausgebildet ist; einer ersten Senke, die wenigstens ein Rechenzentrum mit elektrischen Rechenanlagen zur Nutzung des von der zweiten Quelle bereitgestellten elektrischen Stroms umfasst; einer ersten Kühleinrichtung, die mit der ersten Senke wirkverbunden ist und zur Abfuhr von Abwärme aus der ersten Senke ausgebildet ist; und einer zweiten Senke, die mit der ersten Senke wirkverbunden ist und zur Abfuhr von Abwärme aus der ersten Senke ausgebildet ist.

Die erste Quelle ist vorzugsweise eine lokale Gasquelle, die auf Nutzung von insbesondere in landwirtschaftlichen Betrieben anfallendem Biomasse, insbesondere Bioabfall wie Gülle, Pflanzenabfällen und dergleichen, ausgelegt ist. Das erzeugte Biogas stellt die Energie zur Stromerzeugung in der zweiten Quelle bereit. Die zweite Quelle ist vorzugsweise eine lokale Stromquelle. Die erste Senke ist vorzugsweise eine lokale Senke für elektrischen Strom, insbesondere ein Rechenzentrum.

Mit besonderem Vorteil kann die erste Senke klimaneutral betrieben werden, indem, vorzugsweise ausschließlich, Strom aus regenerativen Quellen in Form von Biogas eingesetzt wird. Beim Betrieb der Vielzahl von Rechenanlagen in der ersten Senke entstehende Abwärme wird nicht in die Umwelt abgegeben, sondern gezielt in der zweiten Senke zur Trocknung und/oder zum Heizen verwendet.

Beispielsweise kann in der zweiten Senke Grasschnitt getrocknet werden, das wiederum als Viehfutter verwendet werden kann und indirekt wieder über daraus generierte Bioabfällen zur Biogaserzeugung oder direkt zur Biogaserzeugung eingesetzt werden kann. Optional kann in der zweiten Senke auch Klärschlamm, Kompost und dergleichen getrocknet werden; dies kann in getrockneter Form weiterverarbeitet werden, insbesondere zur Energieerzeugung genutzt werden.

Optional kann vorgesehen sein, die erste Quelle durch weitere regenerative Stromquellen, wie beispielsweise eine oder mehrere Windkraftanlagen und/oder eine oder mehrere Photovoltaikanlagen und/oder eine oder mehrere Wasserkraftanlagen und/oder eine oder mehrere Erdwärmeanlagen, zu ergänzen. Diese können lokal vorhanden sein oder außerhalb der lokalen Rechenzentrumseinrichtung angeordnet sein. Die eine oder mehreren weiteren Quellen können permanent oder auch temporär an die erste Quelle angekoppelt sein.

Nach einer günstigen Ausgestaltung der Rechenzentrumseinrichtung kann die zweite Senke eine Trocknungsanlage aufweisen, die zur Erzeugung eines Trocknungsprodukts ausgebildet ist, welches direkt oder indirekt der ersten Quelle zur Erzeugung von Biogas zuführbar ist. Es kann ein quasi geschlossener lokaler Energiekreislauf gebildet werden, bei dem elektrischer Strom vor Ort aus vor Ort vorhandenen regenerativen Quellen erzeugt und der ersten Senke zugeführt wird und die dort generierte Abwärme beim Betrieb des wenigstens einen Rechenzentrums vor Ort zur Erzeugung von regenerativem Strom genutzt wird.

Nach einer günstigen Ausgestaltung der Rechenzentrumseinrichtung kann die zweite Senke eine Kopplungsvorrichtung aufweisen, die zur Kopplung an eine oder mehrere weitere Senken, insbesondere ein Fernwärmenetz, ausgebildet ist. Fällt mehr Abwärme an als vor Ort bei der Rechenzentrumseinrichtung durch die zweite Senke verbraucht werden kann, kann überschüssige Abwärme klimafreundlich anderweitig genutzt werden.

Nach einer günstigen Ausgestaltung der Rechenzentrumseinrichtung kann das wenigstens eine Rechenzentrum mit den elektrischen Rechenanlagen der ersten Senke wenigstens einen Zugang für einen Fernzugriff auf ein oder mehrere der Rechenanlagen aufweisen. Vorteilhaft können Nutzer über den Fernzugriff auf die Rechenanlagen zugreifen und diese für eigene Zwecke nutzen.

Nach einer günstigen Ausgestaltung der Rechenzentrumseinrichtung kann der Strombedarf der ersten Senke wenigstens zu 90% aus der zweiten Quelle gedeckt sein. Alternativ oder zusätzliche kann der Wärmebedarf der zweiten Senke wenigstens zu 90% aus der ersten Senke gedeckt sein. Dies erlaubt einen weitgehend autarken lokalen Betrieb der Rechenzentrumseinrichtung.

Nach einer günstigen Ausgestaltung der Rechenzentrumseinrichtung kann das wenigstens eine Rechenzentrum der ersten Senke ausgebildet sein, bedarfsweise temporär, insbesondere örtlich wechselnd, mit wechselnden zweiten Quellen gekoppelt zu sein. Auf diese Weise können örtlich auftretende Energieschwankungen bei der Stromerzeugung ausgeglichen werden. Die erste Senke kann dabei stationär vorgesehen sein oder auch als transportable Einheit. Beispielsweise kann die erste Senke eine oder mehrere Container aufweisen, in denen Rechenanlagen angeordnet sind. Der oder die Container können per LKW transportiert werden und temporär an andere zweite Quellen angeschlossen werden und dort die generierte Abwärme beispielsweise zur Trocknung von Produkten zur Verfügung stellen.

Insbesondere kann die erste Senke der Rechenzentrumseinrichtung eine mobile Einrichtung aufweisen. Günstigerweise kann die erste Senke eine oder mehrere so genannte ISO-Container aufweisen, in denen Rechenanlagen angeordnet sind. Derartige ISO-Container haben Abmessungen, mit denen sie leicht auf einem LKW oder mit der Bahn transportiert werden können. Standardabmessungen derartiger ISO-Container sind eine Breite von 8 Fuß und eine Länge von 20 Fuß oder von 40 Fuß. Weist die erst Senke mehrere Container auf, können einzelne davon temporär an andere Standorte transportiert werden, um dort vorübergehend lokal Strom aufzunehmen und Abwärme abzugeben, aus der letztlich wieder lokal Strom, insbesondere über Biogaserzeugung, erzeugt werden kann. So können unterschiedliche Bedarfe an Stromabnahme und/oder Abwärmeverfügbarkeit vor Ort ausgeglichen werden.

Nach einer günstigen Ausgestaltung der Rechenzentrumseinrichtung kann die Kühleinrichtung ein Gebläse aufweisen. Die Kühleinrichtung kann einfach an die erste Senke angeschlossen werden und die erste Senke, insbesondere die Container, mit einem starken Luftstrom kühlen. Auch bei transportablen mobilen Einrichtungen der ersten Senke kann eine ausreichende Kühlung an anderen Einsatzorten einfach bereitgestellt werden.

Nach einer günstigen Ausgestaltung der Rechenzentrumseinrichtung können die erste und die zweite Quelle in einer Kraft-Wärmekopplungsanlage angeordnet sein. Derartige Kraft-Wärmekopplungsanlagen sind bei landwirtschaftlichen Betrieben häufig verfügbar, so dass die Rechenzentrumseinrichtung vorteilhaft in Kombination mit vorhandenen Einrichtungen von landwirtschaftlichen Betrieben eingesetzt werden kann.

Nach einer günstigen Ausgestaltung der Rechenzentrumseinrichtung können die erste und zweite Quelle und die zweite Senke sowie die Kühleinrichtung Bestandteile eines landwirtschaftlichen Betriebs sein. Vorteilhaft kann eine klimaneutrale, flexible und effiziente Rechenzentrumseinrichtung im ländlichen Bereich bereitgestellt werden. Vorteilhaft kann eine lokale Stromerzeugung genutzt werden, bei der ein häufiges Problem ist, dass im ländlichen Bereich eine ausreichende Anzahl notwendiger Großabnehmer für den aus regenerativen Quellen produzierten Strom fehlen. Dies kann ohne aufwändige Installationen von überregionalen Stromnetzen oder Einspeisepunkten in kommunale Versorgungsnetze durch die lokale Rechenzentrumseinrichtung erfolgen.

Nach einem weiteren Aspekt der Erfindung wird ein Verfahren zum Betreiben einer erfindungsgemäßen Rechenzentrumseinrichtung vorgeschlagen, wobei eine erste Quelle Biogas erzeugt; eine zweite Quelle elektrischen Strom aus dem Biogas erzeugt, die mit der ersten Quelle zusammenwirkt und mit Biogas der ersten Quelle betrieben wird; eine erste Senke mit wenigstens einem Rechenzentrum mit elektrischen Rechenanlagen den von der zweiten Quelle bereitgestellten elektrischen Strom nutzt; eine erste Kühleinrichtung, die mit der ersten Senke zusammenwirkt und Abwärme aus der ersten Senke abführt; und eine zweite Senke, die mit der ersten Senke zusammenwirkt und Abwärme aus der ersten Senke aufnimmt.

Mit besonderem Vorteil kann die erste Senke klimaneutral betrieben werden, indem ausschließlich Strom aus regenerativen Quellen in Form von Biogas eingesetzt wird. Beim Betrieb der Vielzahl von Rechenanlagen in der ersten Senke entstehende Abwärme wird nicht in die Umwelt abgegeben, sondern gezielt in der zweiten Senke zur Trocknung und/oder zum Heizen verwendet.

Es kann ein quasi geschlossener lokaler Energiekreislauf gebildet werden, bei dem elektrischer Strom vor Ort aus vor Ort vorhandenen regenerativen Quellen erzeugt und der ersten Senke zugeführt wird und die dort generierte Abwärme beim Betrieb des wenigstens einen Rechenzentrums vor Ort zur Erzeugung von regenerativem Strom genutzt wird.

Nach einer günstigen Ausgestaltung des Verfahrens kann die zweite Senke eine Trocknungsanlage aufweisen, die ein Trocknungsprodukt erzeugt, welches direkt oder indirekt der ersten Quelle zur Erzeugung von Biogas zuführgeführt werden kann. Es kann ein quasi geschlossener lokaler Energiekreislauf gebildet werden, bei dem elektrischer Strom vor Ort aus vor Ort vorhandenen regenerativen Quellen erzeugt und der ersten Senke zugeführt wird und die dort generierte Abwärme beim Betrieb des wenigstens einen Rechenzentrums vor Ort zur Erzeugung von regenerativem Strom genutzt wird.

Nach einer günstigen Ausgestaltung des Verfahrens kann die zweite Senke mit einer Kopplungsvorrichtung an eine oder mehrere weitere Senken, insbesondere ein Fernwärmenetz, gekoppelt werden. Fällt mehr Abwärme an als vor Ort bei der Rechenzentrumseinrichtung durch die zweite Senke verbraucht werden kann, kann überschüssige Abwärme klimafreundlich anderweitig genutzt werden

Nach einer günstigen Ausgestaltung des Verfahrens kann die erste Senke bedarfsweise temporär, insbesondere örtlich wechselnd, mit wechselnden zweiten Quellen gekoppelt werden. Auf diese Weise können örtlich auftretende Energieschwankungen bei der Stromerzeugung ausgeglichen werden. Die erste Senke kann dabei stationär vorgesehen sein oder auch als transportable Einheit. Beispielsweise kann die erste Senke eine oder mehrere Container aufweisen, in denen Rechenanlagen angeordnet sind. Der oder die Container können per LKW transportiert werden und temporär an anderen zweiten Quellen angeschlossen können und dort die generierte Abwärme beispielsweise zur Trocknung von Produkten zur Verfügung stellen.

Nach einer günstigen Ausgestaltung des Verfahrens kann der Strombedarf der ersten Senke wenigstens zu 90% aus der zweiten Quelle gedeckt werden und/oder der Wärmebedarf der zweiten Senke wenigstens zu 90% aus der ersten Senke gedeckt werden. Dies erlaubt einen weitgehend autarken lokalen Betrieb der Rechenzentrumseinrichtung.

### Zeichnung

Weitere Vorteile ergeben sich aus der folgenden Zeichnungsbeschreibung. In den Zeichnungen sind Ausführungsbeispiele der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen beispielhaft:
- Fig. 1: eine Rechenzentrumseinrichtung nach einem Ausführungsbeispiel der Erfindung, bei dem Abwärme einer ersten Senke zur Trocknung eines Produkts genutzt und das Trocknungsprodukt über eine Zwischenstation einer ersten Quelle wieder zugeführt wird;
- Fig. 2: eine Rechenzentrumseinrichtung nach einem Ausführungsbeispiel der Erfindung, bei dem Abwärme einer ersten Senke zur Trocknung eines Produkts genutzt und das Trocknungsprodukt unmittelbar einer ersten Quelle wieder zugeführt wird;
- Fig. 3: eine Rechenzentrumseinrichtung nach einem Ausführungsbeispiel der Erfindung, bei dem eine erste Senke mehrere Rechenzentren aufweist;
- Fig. 4: eine Rechenzentrumseinrichtung nach einem Ausführungsbeispiel der Erfindung, bei dem eine erste Senke mehrere Rechenzentren aufweist, von denen wenigstens eines mobil ist;
- Fig. 5: eine Rechenzentrumseinrichtung nach einem Ausführungsbeispiel der Erfindung, bei dem eine erste Senke temporär mit einer externen ersten, Strom produzierenden Quelle gekoppelt ist;
- Fig. 6: ein Flussdiagramm eines Verfahrens zum Betreiben einer Rechenzentrumseinrichtung nach einem Ausführungsbeispiel der Erfindung.

### Ausführungsformen der Erfindung

In den Figuren sind gleichartige oder gleichwirkende Komponenten mit gleichen Bezugszeichen beziffert. Die Figuren zeigen lediglich Beispiele und sind nicht beschränkend zu verstehen.

Bevor die Erfindung im Detail beschrieben wird, ist darauf hinzuweisen, dass sie nicht auf die jeweiligen Bauteile der Vorrichtung sowie die jeweiligen Verfahrensschritte beschränkt ist, da diese Bauteile und Verfahren variieren können. Die hier verwendeten Begriffe sind lediglich dafür bestimmt, besondere Ausführungsformen zu beschreiben und werden nicht einschränkend verwendet. Wenn zudem in der Beschreibung oder in den Ansprüchen die Einzahl oder unbestimmte Artikel verwendet werden, bezieht sich dies auch auf die Mehrzahl dieser Elemente, solange nicht der Gesamtzusammenhang eindeutig etwas Anderes deutlich macht.

Im Folgenden verwendete Richtungsterminologie mit Begriffen wie "links", "rechts", "oben", "unten", "davor" "dahinter", "danach" und dergleichen dient lediglich dem besseren Verständnis der Figuren und soll in keinem Fall eine Beschränkung der Allgemeinheit darstellen. Die dargestellten Komponenten und Elemente, deren Auslegung und Verwendung können im Sinne der Überlegungen eines Fachmanns variieren und an die jeweiligen Anwendungen angepasst werden.

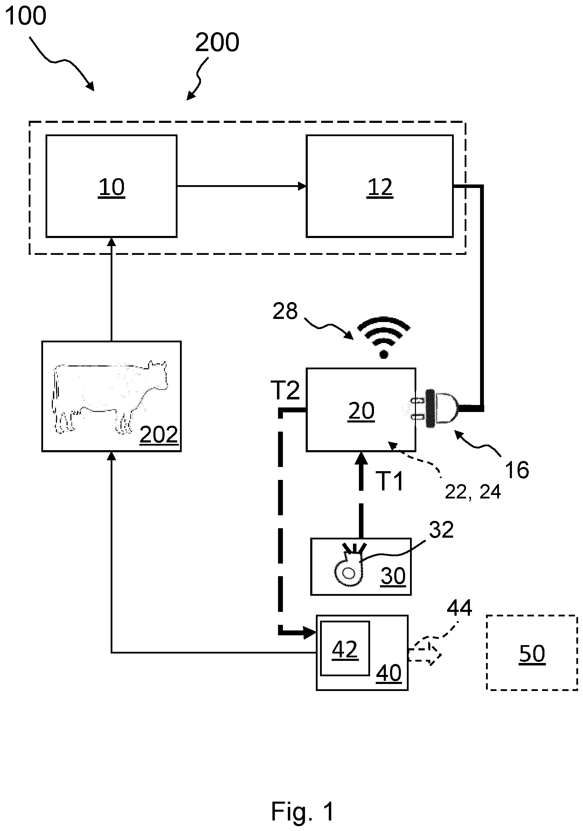

Figur 1 zeigt eine Rechenzentrumseinrichtung 100 nach einem Ausführungsbeispiel der Erfindung, bei dem Abwärme einer ersten Senke 20 zur Trocknung eines Produkts genutzt und das Trocknungsprodukt über eine Zwischenstation 202 einer ersten Quelle 10 wieder zugeführt wird. Die Rechenzentrumseinrichtung 100 ist vorzugsweise in einem landwirtschaftlichen Betrieb 200 angeordnet und umfasst dortige Komponenten zur Energieaufnahme in Form von elektrischem Strom und Energieabgabe in Form von Abwärme.

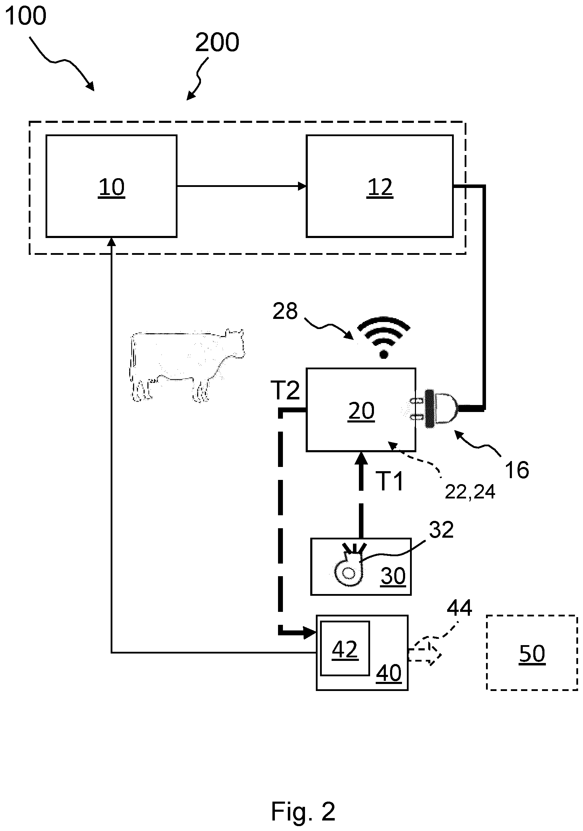

Figur 2 zeigt eine Rechenzentrumseinrichtung 100 nach einem Ausführungsbeispiel der Erfindung, bei dem Abwärme der ersten Senke 20 zur Trocknung eines Produkts genutzt und das Trocknungsprodukt unmittelbar der ersten Quelle 10 wieder zugeführt wird, ohne Umweg über eine Nutzungseinrichtung.

Die Rechenzentrumseinrichtung 100 umfasst eine erste Quelle 10 zur Erzeugung von Biogas, sowie eine zweite Quelle 12 zur Erzeugung von elektrischem Strom aus dem Biogas, die mit der ersten Quelle 10 wirkverbunden ist und zum Betreiben mit Biogas der ersten Quelle 10 ausgebildet ist. Die erste und die zweite Quelle 10, 12 können beispielsweise in einer Kraft-Wärmekopplungsanlage kombiniert sein.

Eine erste Senke 20 umfasst wenigstens ein Rechenzentrum 24 mit elektrischen Rechenanlagen 22 zur Nutzung des von der zweiten Quelle 12 bereitgestellten elektrischen Stroms. Die erste Senke 20 wird von einer ersten Kühleinrichtung 30 gekühlt, die mit der ersten Senke 20 wirkverbunden ist und zur Abfuhr von Abwärme aus der ersten Senke 20 ausgebildet ist.

Eine zweite Senke 40 ist an die erste Senke 20 angeschlossen und mit der ersten Senke 20 wirkverbunden und zur Abfuhr von Abwärme aus der ersten Senke 20 ausgebildet.

Beispielsweise stellt die Kühleinrichtung 30 über ein Gebläse 32 einen Luftstrom zur Abfuhr von Abwärme aus der ersten Senke 20 bereit. Der Luftstrom ist in der Figur durch einen dicken unterbrochenen Pfeil angedeutet. Das Gebläse kann einfach über einen geeigneten Schlauch an die erste Senke 20 angeschlossen werden, und der mit der Abwärme erhitzte Luftstrom kann über einen weiteren Schlauch der zweiten Senke 40 zugeführt werden. Kühle Luft wird mit einer Temperatur T1 in die erste Senke 20 eingespeist und erhitzte Luft wird mit der Temperatur T2 aus der erste Senke 20 abgeführt.

Die zweite Senke 40 weist eine Trocknungsanlage 42 auf, die zur Erzeugung eines Trocknungsprodukts ausgebildet ist. Die Abwärme der ersten Senke 20 wird in der zweiten Senke 40 beispielsweise zur Trocknung von Grasschnitt zu Heu verwendet, das im Ausführungsbeispiel der Figur 1 als Futter für Vieh in der Nutzungseinrichtung 202 bereitgestellt wird. Das Futter wird dort in Biomasse, insbesondere Bioabfall wie Dung und dergleichen, umgesetzt, welcher der ersten Quelle 10 zur Biogaserzeugung zugeführt wird. Alternativ wird das Trocknungsprodukt im Ausführungsbeispiel der Figur 2 direkt von der Trocknungsanlage 42 zur ersten Quelle 10 zur Erzeugung von Biogas zugeführt.

Optional kann die zweite Senke 40 eine Kopplungsvorrichtung 44 aufweisen, die zur Kopplung an eine oder mehrere weitere Senken 50, insbesondere ein Fernwärmenetz, ausgebildet ist, falls mehr Abwärme in der ersten Senke 20 anfällt als in der zweiten Senke 40 benötigt wird.

Die erste Senke 20 umfasst eine oder mehrere Rechenzentren 24 mit elektrischen Rechenanlagen 22 und weist wenigstens einen Zugang 28 für einen Fernzugriff auf das Rechenzentrum 24 oder die mehreren Rechenzentren 22 auf, etwa einen WLAN-Anschluss für eine drahtlose Internetverbindung. Hierüber können Nutzer auf die Rechenanlagen 22 der ersten Senke 20 zugreifen und dort Rechenoperationen durchführen.

Der Strombedarf der ersten Senke 20 wird wenigstens zu 90% aus der zweiten Quelle 12 gedeckt ist. Ebenso wird der Wärmebedarf der zweiten Senke 40 wenigstens zu 90% aus der ersten Senke 20 gedeckt. Hierdurch ist ein lokaler quasi geschlossener Energiekreislauf möglich, bei dem die benötigte elektrische Leistung für das oder die Rechenzentren 24 der ersten Senke 20 mit den elektrischen Rechenanlagen 22 lokal aus regenerativen Quellen in Form von Biogas erzeugt wird und bei Betrieb der Rechenanlagen 22 erzeugte Abwärme genutzt wird, um einen Rohstoff für die Biogaserzeugung in der ersten Quelle 10 zu liefern, mit dem dann wieder Strom in der zweiten Quelle 12 erzeugt wird.

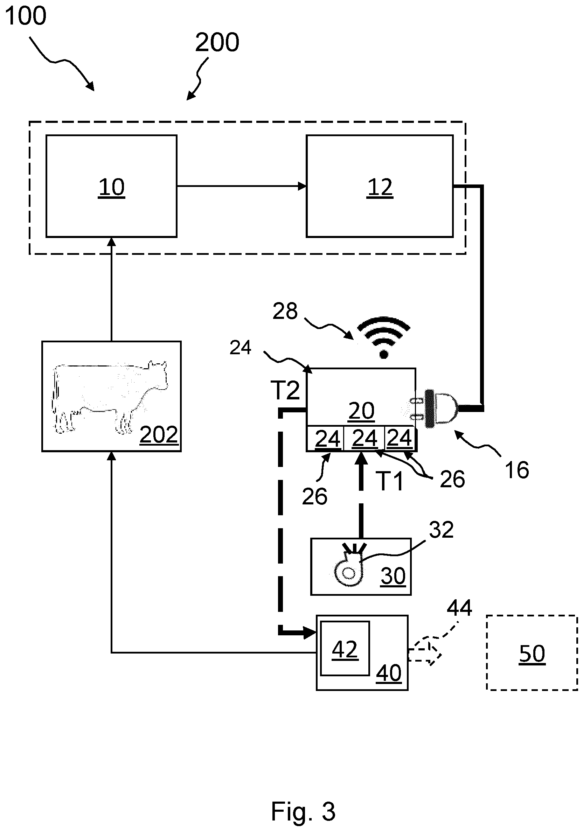

Figur 3 zeigt eine Rechenzentrumseinrichtung 100 nach einem Ausführungsbeispiel der Erfindung, das eine erste Senke 20 mehrere Rechenzentren 24 aufweist. Die weiteren Elemente der Rechenzentrumseinrichtung 100 entsprechen den vorangegangenen Ausführungsbeispielen, auf die zur Vermeidung unnötiger Wiederholungen verwiesen wird.

Die erste Senke 20 weist die Rechenzentren 24 als mobile Einheiten 26 auf. Diese können vorteilhaft als ISO-Container ausgebildet sein, die als Standardcontainer leicht auf LKWs transportiert werden können.

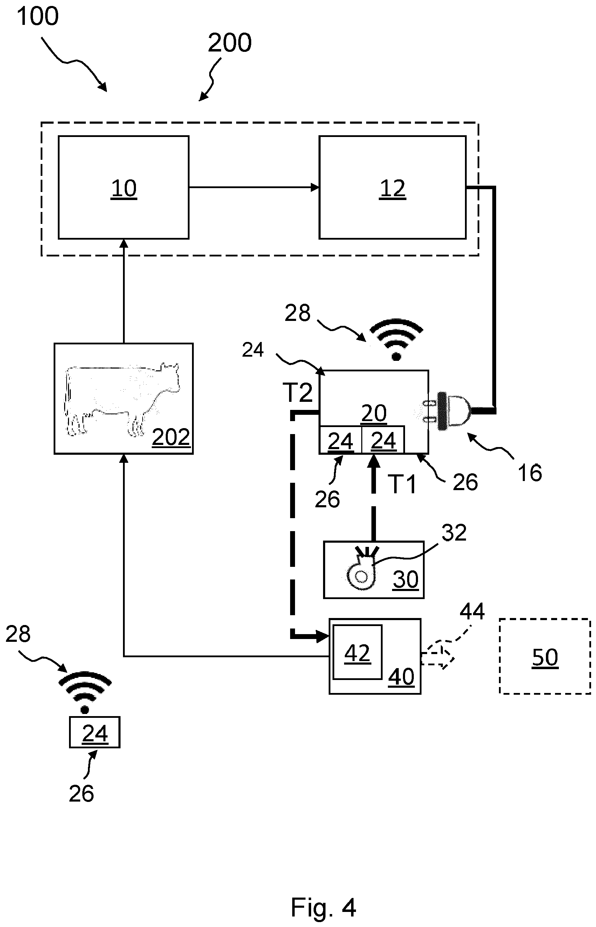

Im Ausführungsbeispiel der Figur 4 ist eines der Rechenzentren 24 der ersten Senke 20 aus Figur 3 temporär an einem von der lokalen Rechenzentrumseinrichtung 100 entfernten Ort eingesetzt. Dies kann ein weiterer landwirtschaftlicher Betrieb sein, bei dem vorzugsweise ebenso eine Stromversorgung mittels regenerativ erzeugtem Strom erfolgt, insbesondere mittels lokal erzeugtem Biogas, und Abwärme des einen Rechenzentrums 24 lokal am anderen Ort genutzt wird, um in einer dortigen Trocknungsanlage 42 ein Trocknungsprodukt, beispielsweise Heu als Viehfutter, zu erzeugen. Das entfernt angeordnete Rechenzentrum 24 ist mit einem eigenen Zugang 28 für einen Fernzugriff auf die Rechenanlagen des Rechenzentrums 24 ausgestattet.

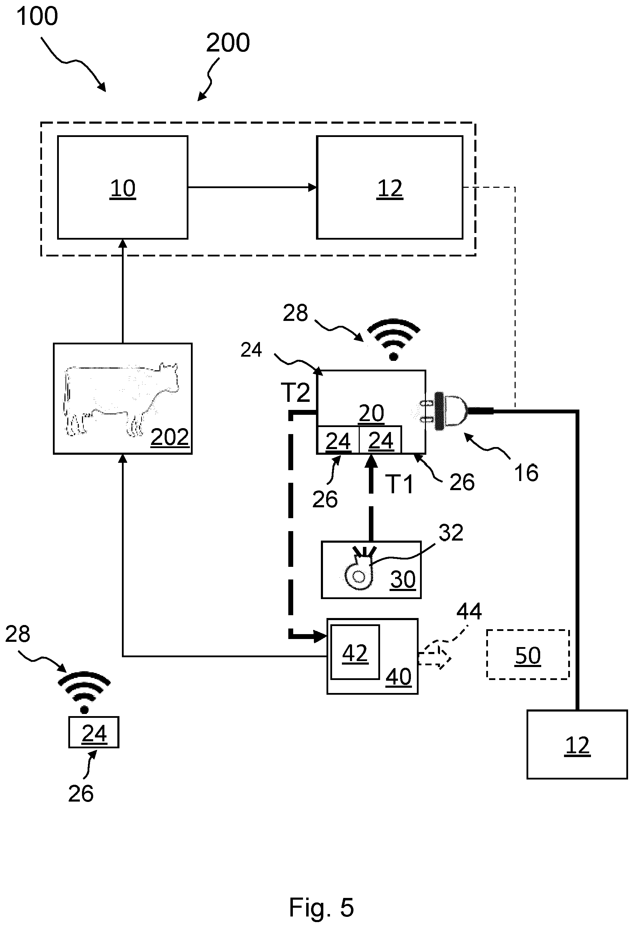

Figur 5 zeigt eine Rechenzentrumseinrichtung 100 nach einem Ausführungsbeispiel der Erfindung, bei dem eine erste Senke 20 temporär mit einer externen ersten, Strom produzierenden Quelle gekoppelt ist. Die weiteren Elemente der Rechenzentrumseinrichtung 100 entsprechen den vorangegangenen Ausführungsbeispielen, auf die zur Vermeidung unnötiger Wiederholungen verwiesen wird.

In diesem Ausführungsbeispiel ist nicht nur eines der als mobile Einheiten 26 ausgebildeten Rechenzentren 24 temporär entfernt von der lokalen Rechenzentrumseinrichtung 100 eingesetzt. Ebenso ist die erste Senke 20 mit den restlichen Rechenzentren 24 zur Stromversorgung der ersten Senke 20 temporär an eine externe zweite Quelle 12 angeschlossen. Diese kann am gleichen Ort wie das temporär extern angeordnete Rechenzentrum 24 vorhanden sein oder an einem separaten Ort.

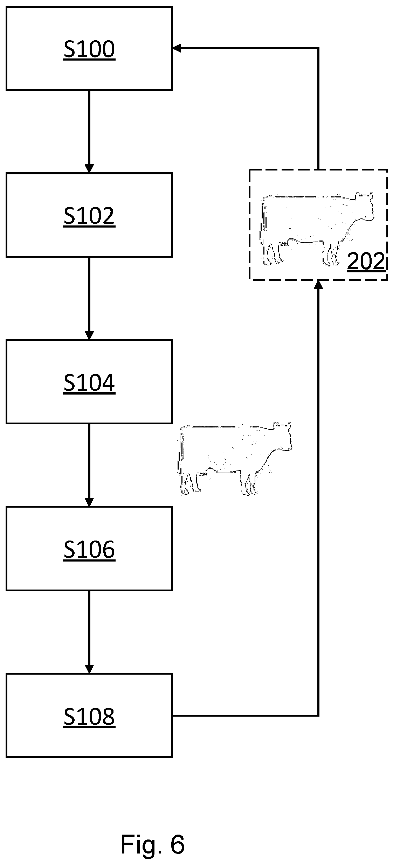

Figur 6 zeigt ein Flussdiagramm eines Verfahrens zum Betreiben einer lokalen Rechenzentrumseinrichtung 100 nach einem Ausführungsbeispiel der Erfindung, welche lokal vorhandene Komponenten eines landwirtschaftlichen Betriebs 200 (Figuren 1-5) umfasst.

In Schritt S100 erzeugt eine erste Quelle 10 Biogas, das aus in dem landwirtschaftlichen Betrieb 200 anfallenden Rohstoffen erzeugt wird.

In Schritt S102 erzeugt eine zweite Quelle 12 elektrischen Strom aus dem Biogas, das in der ersten Quelle 10 erzeugt wird.

In Schritt S104 nutzt eine erste Senke 20 mit elektrischen Rechenanlagen 22 den von der zweiten Quelle 12 bereitgestellten elektrischen Strom.

In Schritt S106 liefert eine erste Kühleinrichtung 30 Kühlung für die erste Senke 20 und führt Abwärme aus der ersten Senke 20 ab.

In Schritt S108 nimmt eine zweite Senke 40, die mit der ersten Senke 20 zusammenwirkt, Abwärme aus der ersten Senke 20 auf. Die zweite Senke 40 weist eine Trocknungsanlage 42 auf, die ein Trocknungsprodukt, beispielsweise Heu, erzeugt, welches direkt oder indirekt der ersten Quelle 10 zur Erzeugung von Biogas zuführgeführt wird, womit das Verfahren wieder mit Schritt S100 beginnt.

Es kann jeweils eine Abfrage erfolgen, ob ausreichend Strom zur Verfügung steht, um die erste Senke 20 zu versorgen. Falls nein, kann optional Strom aus einer externen ersten Quelle geliefert werden. Ebenso kann alternativ oder zusätzlich eines oder mehrere der Rechenzentren der ersten Quelle 10 temporär an einen oder mehrere andere Standorte transportiert und dort betrieben werden.

Ebenso kann eine Abfrage erfolgen, ob in der ersten Senke 20 genug Abwärme erzeugt wird, um die Trocknungsanlage 42 der zweiten Senke 40 zu betreiben. Ferner kann eine Abfrage erfolgen, ob in der ersten Senke 20 zu viel Abwärme für die Trocknungsanlage 42 erzeugt wird. Falls ja, kann die zweite Senke 40 mit einer Kopplungsvorrichtung 44 temporär an eine oder mehrere weitere Senken 50, insbesondere ein Fernwärmenetz, gekoppelt werden.
- 10: erste Quelle
- 12: zweite Quelle
- 16: elektrische Kopplung
- 20: erste Senke
- 22: Rechenanlage
- 24: Rechenzentrum
- 26: mobile Einrichtung
- 28: Zugang
- 30: erste Kühleinrichtung
- 32: Gebläse
- 34: Kopplungsvorrichtung
- 40: zweite Senke
- 42: Trocknungsanlage
- 44: Kopplungsvorrichtung
- 50: weitere Senke
- 100: Rechenzentrumseinrichtung
- 200: landwirtschaftlicher Betrieb
- 202: Nutzungseinrichtung

## Patentansprüche

1. Rechenzentrumseinrichtung (100) mit
(a) einer ersten Quelle (10) zur Erzeugung von Biogas,
(b) einer zweiten Quelle (12) zur Erzeugung von elektrischem Strom aus dem Biogas, die mit der ersten Quelle (10) wirkverbunden ist und zum Betreiben mit Biogas der ersten Quelle (10) ausgebildet ist,
(c) einer ersten Senke (20), die wenigstens ein Rechenzentrum (24) mit elektrischen Rechenanlagen (22) zur Nutzung des von der zweiten Quelle (12) bereitgestellten elektrischen Stroms umfasst,
(d) einer ersten Kühleinrichtung (30), die mit der ersten Senke (20) wirkverbunden ist und zur Abfuhr von Abwärme aus der ersten Senke (20) ausgebildet ist,
(e) einer zweiten Senke (40), die mit der ersten Senke (20) wirkverbunden ist und zur Abfuhr von Abwärme aus der ersten Senke (20) ausgebildet ist.

2. Rechenzentrumseinrichtung nach Anspruch 1, **dadurch gekennzeichnet, dass** die zweite Senke (40) eine Trocknungsanlage (42) aufweist, die zur Erzeugung eines Trocknungsprodukts ausgebildet ist, welches direkt oder indirekt der ersten Quelle (10) zur Erzeugung von Biogas zuführbar ist.

3. Rechenzentrumseinrichtung nach Anspruch 1 oder 2, **dadurch gekennzeichnet, dass** die zweite Senke (40) eine Kopplungsvorrichtung (44) aufweist, die zur Kopplung an eine oder mehrere weitere Senken (50), insbesondere ein Fernwärmenetz, ausgebildet ist.

4. Rechenzentrumseinrichtung nach einem der vorhergehenden Ansprüche, **dadurch gekennzeichnet, dass** das wenigstens eine Rechenzentrum (24) mit elektrischen Rechenanlagen (22) der ersten Senke (20) wenigstens einen Zugang (28) für einen Fernzugriff auf eine oder mehrere der Rechenanlagen (22) aufweist.

5. Rechenzentrumseinrichtung nach einem der vorhergehenden Ansprüche, **dadurch gekennzeichnet, dass** der Strombedarf der ersten Senke (20) wenigstens zu 90% aus der zweiten Quelle (12) gedeckt ist und/oder dass der Wärmebedarf der zweiten Senke (40) wenigstens zu 90% aus der ersten Senke (20) gedeckt ist.

6. Rechenzentrumseinrichtung nach einem der vorhergehenden Ansprüche, **dadurch gekennzeichnet, dass** das wenigstens eine Rechenzentrum (24) der ersten Senke (20) ausgebildet ist, bedarfsweise temporär, insbesondere örtlich wechselnd, mit wechselnden zweiten Quellen (12) gekoppelt zu sein.

7. Rechenzentrumseinrichtung nach Anspruch 6, **dadurch gekennzeichnet, dass** die erste Senke (20) eine mobile Einrichtung (26) aufweist.

8. Rechenzentrumseinrichtung nach einem der vorhergehenden Ansprüche, **dadurch gekennzeichnet, dass** die Kühleinrichtung (30) ein Gebläse (32) aufweist.

9. Rechenzentrumseinrichtung nach einem der vorhergehenden Ansprüche, **dadurch gekennzeichnet, dass** die erste und die zweite Quelle (10, 12) in einer Kraft-Wärmekopplungsanlage angeordnet sind.

10. Rechenzentrumseinrichtung nach einem der vorhergehenden Ansprüche, **dadurch gekennzeichnet, dass** die erste und zweite Quelle (10, 12) und die zweite Senke (40) sowie die Kühleinrichtung (30) Bestandteile eines landwirtschaftlichen Betriebs (200) sind.

11. Verfahren zum Betreiben einer Rechenzentrumseinrichtung (100) nach einem der Ansprüche 1 bis 10, wobei
(a) eine erste Quelle (10) Biogas erzeugt,
(b) eine zweite Quelle (12) elektrischen Strom aus dem Biogas erzeugt, die mit der ersten Quelle (10) zusammenwirkt und mit Biogas der ersten Quelle (10) betrieben wird,
(c) eine erste Senke (20) mit wenigstens einem Rechenzentrum (24) mit elektrischen Rechenanlagen (22) den von der zweiten Quelle (12) bereitgestellten elektrischen Strom nutzt,
(d) eine erste Kühleinrichtung (30), die mit der ersten Senke (20) zusammenwirkt und Abwärme aus der ersten Senke (20) abführt,
(e) eine zweite Senke (40), die mit der ersten Senke (20) zusammenwirkt und Abwärme aus der ersten Senke (20) aufnimmt.

12. Verfahren nach Anspruch 11, **dadurch gekennzeichnet, dass** die zweite Senke (40) eine Trocknungsanlage (42) aufweist, die ein Trocknungsprodukt erzeugt, welches direkt oder indirekt der ersten Quelle (10) zur Erzeugung von Biogas zuführgeführt wird.

13. Verfahren nach Anspruch 11 oder 12, **dadurch gekennzeichnet, dass** die zweite Senke (40) mit einer Kopplungsvorrichtung (44) an eine oder mehrere weitere Senken (50), insbesondere ein Fernwärmenetz, gekoppelt wird.

14. Verfahren nach einem der Ansprüche 11 bis 13, **dadurch gekennzeichnet, dass** die erste Senke (20) bedarfsweise temporär, insbesondere örtlich wechselnd, mit wechselnden zweiten Quellen (12) gekoppelt wird.

15. Verfahren nach einem der Ansprüche 11 bis 14, **dadurch gekennzeichnet, dass** der Strombedarf der ersten Senke (20) wenigstens zu 90% aus der zweiten Quelle (12) gedeckt wird und/oder dass der Wärmebedarf der zweiten Senke (40) wenigstens zu 90% aus der ersten Senke (20) gedeckt wird.
